# EUROPEAN PATENT SPECIFICATION

(11) **EP 3 137 594 B1**
(45) Date of publication and mention of the grant of the patent: **28.03.2018**
(21) Application number: 15721340.6
(22) Date of filing: 30.04.2015
(51) Int. Cl.: C12N 5/00, C12N 5/071

(54) **METHOD FOR PRODUCING A TOTALLY ENDOGENOUS BIOENGINEERED TISSUE AND TISSUE OBTAINED THEREBY**
VERFAHREN ZUR HERSTELLUNG EINES VOLLSTÄNDIG ENDOGENEN GENMANIPULIERTEN GEWEBE UND DAMIT HERGESTELLTES GEWEBE
PROCÉDÉ DE PRODUCTION D'UN TISSU TOTALEMENT ENDOGÈNE PRODUIT PAR GÉNIE BIOLOGIQUE ET TISSU AINSI OBTENU

(30) Priority: 30.04.2014 US 201461986627 P
(43) Date of publication of application: 08.03.2017
(73) Proprietor: Fondazione Istituto Italiano di Tecnologia, 16163 Genova (IT)
(72) Inventor: IMPARATO, Giorgia, I-16163 Genova (IT); CASALE, Costantino, I-16163 Genova (IT); URCIUOLO, Francesco, I-16163 Genova (IT); NETTI, Paolo, I-16163 Genova (IT); SCAMARDELLA, Sara, I-16163 Genova (IT)
(74) Representative: Bosia, Alessandra
(86) International application number: PCT/IB2015/053166
(87) International publication number: WO 2015/166455

(56) References cited:
- CASALEC., IMPARATO G., URCIUOLO F., NETTI P.A.: "Realization a complex skin equivalent tissue", J.INVEST. DERMATOL., vol. 134, 10 September 2014 (2014-09-10), page s50, XP009184833,
- LLAMES S G ET AL: "Human plasma as a dermal scaffold for the generation of a completely autologous bioengineered skin", TRANSPLANTATION, WILLIAMS AND WILKINS, GB, vol. 77, no. 3, 1 February 2004 (2004-02-01), pages 350-355, XP002428654, ISSN: 0041-1337, DOI: 10.1097/01.TP.0000112381.80964.85
- STARK H J ET AL: "Authentic fibroblast matrix in dermal equivalents normalises epidermal histogenesis and dermo-epidermal junction in organotypic co-culture", EUROPEAN JOURNAL OF CELL BIOLOGY, WISSENSCHAFLICHE VERLAGSGESELLSCHAFT, STUTTGART, DE, vol. 83, no. 11-12, 1 January 2004 (2004-01-01), pages 631-645, XP004954715, ISSN: 0171-9335, DOI: 10.1078/0171-9335-00435
- SYLVIANE GUERRET ET AL: "Long-term remodeling of a bilayered living human skin equivalent (Apligraf ) grafted onto nude mice: immunolocalization of human cells and characterization of extracellular matrix", WOUND REPAIR AND REGENERATION, vol. 11, no. 1, 1 January 2003 (2003-01-01), pages 35-45, XP055196402, ISSN: 1067-1927, DOI: 10.1046/j.1524-475X.2003.11107.x
- BOEHNKE ET AL: "Effects of fibroblasts and microenvironment on epidermal regeneration and tissue function in long-term skin equivalents", EUROPEAN JOURNAL OF CELL BIOLOGY, WISSENSCHAFLICHE VERLAGSGESELLSCHAFT, STUTTGART, DE, vol. 86, no. 11-12, 13 November 2007 (2007-11-13), pages 731-746, XP022342142, ISSN: 0171-9335, DOI: 10.1016/J.EJCB.2006.12.005
- F. URCIUOLO ET AL: "Fabrication of 3D tissue equivalent: an in vitro platform for understanding collagen evolution in healthy and diseased models", PROCEEDINGS OF SPIE, vol. 8792, 23 May 2013 (2013-05-23), page 87920K, XP055195884, ISSN: 0277-786X, DOI: 10.1117/12.2022119
- IMPARATO GIORGIA ET AL: "The role of microscaffold properties in controlling the collagen assembly in 3D dermis equivalent using modular tissue engineering", BIOMATERIALS, vol. 34, no. 32, 24 July 2013 (2013-07-24) , pages 7851-7861, XP028686801, ISSN: 0142-9612, DOI: 10.1016/J.BIOMATERIALS.2013.06.062
- FRANCESCO URCIUOLO ET AL: "Building a Tissue In Vitro from the Bottom Up: Implications in Regenerative Medicine", METHODIST DEBAKEY CARDIOVASCULAR JOURNAL, vol. 9, no. 4, 1 October 2013 (2013-10-01) , pages 213-217, XP55195307, ISSN: 1947-6094, DOI: 10.14797/mdcj-9-4-213
- PALMIERO C ET AL: "Engineered dermal equivalent tissue in vitro by assembly of microtissue precursors", ACTA BIOMATERIALIA, ELSEVIER, AMSTERDAM, NL, vol. 6, no. 7, 1 July 2010 (2010-07-01), pages 2548-2553, XP027052685, ISSN: 1742-7061 [retrieved on 2010-01-25]
- FRANCESCO URCIUOLO ET AL: "Effect of Process Conditions on the Growth of Three-Dimensional Dermal-Equivalent Tissue Obtained by Microtissue Precursor Assembly", TISSUE ENGINEERING PART C: METHODS, vol. 17, no. 2, 1 February 2011 (2011-02-01), pages 155-164, XP55195301, ISSN: 1937-3384, DOI: 10.1089/ten.tec.2010.0355
- JENNIFER S. LIU ET AL: "Directing the assembly of spatially organized multicomponent tissues from the bottom up", TRENDS IN CELL BIOLOGY, vol. 22, no. 12, 1 December 2012 (2012-12-01), pages 683-691, XP55195701, ISSN: 0962-8924, DOI: 10.1016/j.tcb.2012.09.004
- HOUYONG LUO ET AL: "Fabrication of viable centimeter-sized 3D tissue constructs with microchannel conduits for improved tissue properties through assembly of cell-laden microbeads", JOURNAL OF TISSUE ENGINEERING AND REGENERATIVE MEDICINE, vol. 8, no. 6, 3 July 2012 (2012-07-03), pages 493-504, XP55195293, ISSN: 1932-6254, DOI: 10.1002/term.1554
- XIU WANG ET AL: "Perfusion culture-induced template-assisted assembling of cell-laden microcarriers is a promising route for fabricating macrotissues", BIOTECHNOLOGY JOURNAL, vol. 9, no. 11, 6 October 2014 (2014-10-06), pages 1425-1434, XP055195286, ISSN: 1860-6768, DOI: 10.1002/biot.201400238
- MEI Y ET AL: "Modulating and modeling aggregation of cell-seeded microcarriers in stirred culture system for macrotissue engineering", JOURNAL OF BIOTECHNOLOGY, ELSEVIER SCIENCE PUBLISHERS, AMSTERDAM, NL, vol. 150, no. 3, 1 November 2010 (2010-11-01), pages 438-446, XP027484014, ISSN: 0168-1656, DOI: 10.1016/J.JBIOTEC.2010.09.953 [retrieved on 2010-10-01]

## Description

### TECHNICAL FIELD

The present invention relates to a method for producing a totally endogenous bioengineered tissue including a first layer of connective tissue and a second layer of epithelial tissue, as well as to a tissue obtained by the method and to a method for determining the effect of a chemical substance or an agent on skin.

### BACKGROUND OF THE INVENTION

Skin, the largest organ in the body, performs many important roles, such as barrier protection from physical or chemical insults, sensory functions, and regulation of homeostasis. Skin is composed of two main layers: the surface epithelium or epidermis, which contains keratinocytes as one type of epidermal cells, and the subjacent connective tissue layer or dermis, which contains fibroblasts as one type of dermal cells. Cross-talking between dermis and epidermis layers regulates skin homeostasis. As a consequence, an alteration, at cellular or extracellular level, of one of the two layers can affect the other one. For example, UV irradiation induces epidermal keratinocytes to secrete substances, which stimulate MMP-1 synthesis in dermal fibroblasts, thereby inducing alteration in dermis structure.

To date several human skin equivalent (HSE) models have been developed. EP0020753, EP0418035, US20070269792, US5861153 and US20090298042 disclose human skin equivalents obtained by depositing human keratinocytes on a support, often a dermis equivalent, and cultured so as to lead to differentiation of an epidermis equivalent. The dermis equivalent can consists of spongy, cross-linked material that, in addition to collagen, also may contain other non-skin-specific materials. As an alternative, non-human dermal equivalents e.g. collagen originating from rat or bovine tendon and bovine fibrin mixed with other skin specific protein and fibroblasts can be used.

These non-human biopolymers allow for successful HSE formation, but render the dermal compartment an incomplete approach to human ECM in vivo, because they lead to the formation of an exogenous scaffold (i.e. collagen not synthesized by fibroblast) that needs to be seeded with fibroblasts in order to provide a microenvironment allowing HSE formation, thereby increasing their artificial character. When fibroblast sheets are used as dermal equivalent, an endogenous ECM is present but the cell content is higher than that found in native dermis. As a consequence, native ECM organization cannot be reproduced and only a very small thickness can be obtained.

Human skin equivalents as similar to native skin as possible are of paramount importance in order to be used both as a substitute in case of injured skin and as test skin in dermatology and in allergology for testing substances, for example, potential medicaments or cosmetics, or agents, such as light and heat, for their pharmacological or cosmetic effects, more particularly irritation, toxicity and inflammation effects, and for their compatibility. In addition, such systems can be used for various immunological, histological and molecular-biological tasks. These include, for example, studies on wound healing and studies on the penetration and absorption of substances. The testing of substances using such whole skin models affords major advantages over animal tests and tests with human volunteers because the results obtained with whole skin models are more reproducible and the tests can be carried out more quickly and at less cost. More importantly, the use of such skin models, in place of animal experiments, is in accordance with the commitments of the European Partnership for Alternative Approaches to Animal Testing (EPAA) that aims at pooling knowledge and resources to accelerate the development, validation and acceptance of alternative approaches to further reduce, refine and replace animal use in regulatory testing.

The human skin equivalent models available up to now do not allow reliable results due to the fact that they are not similar enough to native skin and contain a considerable exogenous portion. In particular, studies requiring an endogenous ECM (for example those assessing the effect of UV radiation in the impairment of collagen and elastin function in skin) cannot be carried out efficiently on prior art HSE models.

LLames SG et al. (Transplantation (2004), 77(3): 350-5) discloses the generation of an autologous skin equivalent by seeding keratinocytes onto a dermal equivalent obtained from fibroblasts seeded onto a plasma-based scaffold.

Imparato G et al (Biomaterials (2013) 34(32): 7851-61) and Palmiero C et al. (Acta Biomaterialia (2010) 6(7): 2548-2553) disclose the production of a dermis equivalent by seeding fibroblasts onto microcarriers and microtissue precursors.

### DISCLOSURE OF INVENTION

An object of the present invention is to produce a totally endogenous bioengineered issue, in particular a whole skin model substantially corresponding to native skin, which has a dermis layer and an epidermis layer, avoids the above mentioned disadvantages of the prior art and can be used as test skin for studying pharmacological and cosmetic effects.

This object is achieved by the present invention as it relates to a method as defined in claim 1.

It is a further object of the present invention to provide a method for determining the effect of a chemical substance or an agent on skin as defined in claim 9.

### DEFINITIONS

Unless defined otherwise, all technical and scientific terms used herein have the same meaning as commonly understood by one of ordinary skill in the art to which this invention pertains.

By the term "totally endogenous bioengineered tissue", there is intended a biological tissue obtained by means of in vitro culturing of eukaryotic cells of human origin.

By the term "microtissue" there is intended a complex consisting of a microcarrier surrounded by eukaryotic cells and a micrometric layer of their extracellular matrix.

By the term "dermis equivalent" there is intended a totally endogenous bioengineered dermis that may be used in substitution of native dermis.

By the term "skin equivalent" there is intended a totally endogenous bioengineered skin, comprising a dermal layer and an epidermal layer, which may be used in substitution of native skin, for example for testing substances or agents such as light and heat for their effects.

By the term "epidermal cells" there is intended keratinocytes and melanocytes that can derive from any origin, but are preferably cells of human origin prepared from dissociated human epidermis from foreskin or breast biopsy.

### BRIEF DESCRIPTION OF THE DRAWINGS

Figure 1 shows the maturation chamber used in the method of the present invention, designed to obtain a large endogenous dermis equivalent on the left, and a picture of the endogenous dermis equivalent obtained by the method (25 cm²) in size on the right.
Figure 2 shows the mechanical properties of the human endogenous dermis equivalent. The stress-curve is reported at two deformation rates (5 mm/min and 10 mm/min).
Figure 3 shows a second harmonic generation image of the collagen bundle in the endogenous ECM of the dermis equivalent (scale bar 40 *µ*m).
Figure 4 shows the evolution of a wound healing process. "Wounded" endogenous dermis equivalent is shown in Figure 4A. The complete closure of the wound area after 3 days is shown in Figure 4B. The cells close the wound at a rate of 0.3 mm²/day (scale bar 200 *µ*m).
Figure 5 shows the epidermal cell seeding on the top of dermal equivalent. Left: epidermal cells are seeded drop by drop on the dermal equivalent surface. Centre: histology of the longitudinal section stained with hematoxylin and eosin show the full the human skin equivalent morphology. Right: human skin equivalent surface show brown spot indicating that pigmentation has occurred.
Figure 6 shows a longitudinal section of the skin equivalent that highlights the presence of melanocytes and its pigment of melanin by histochemical analysis such as Fontana Masson.
Figure 7 shows the dermis-epidermis junction in the skin model observed by electron transmission microscopy (scale bar 1 *µ*m). The inset shows a higher magnification (scale bar 500 nm).
Figure 8 shows a histology of a longitudinal section of the skin equivalent made of both dermis and epidermis and devoid of exogenous extracellular matrix stained by hematoxylin and eosin. The basal membrane has characteristic rete ridge structures with typical epidermis appendages going deep through the underlying dermis resembling bulge-like structure. These invaginations are delimited by a basal membrane similar to the vitrea membrane delimiting the hair bulge in the native human skin (scale bar 100 *µ*m).

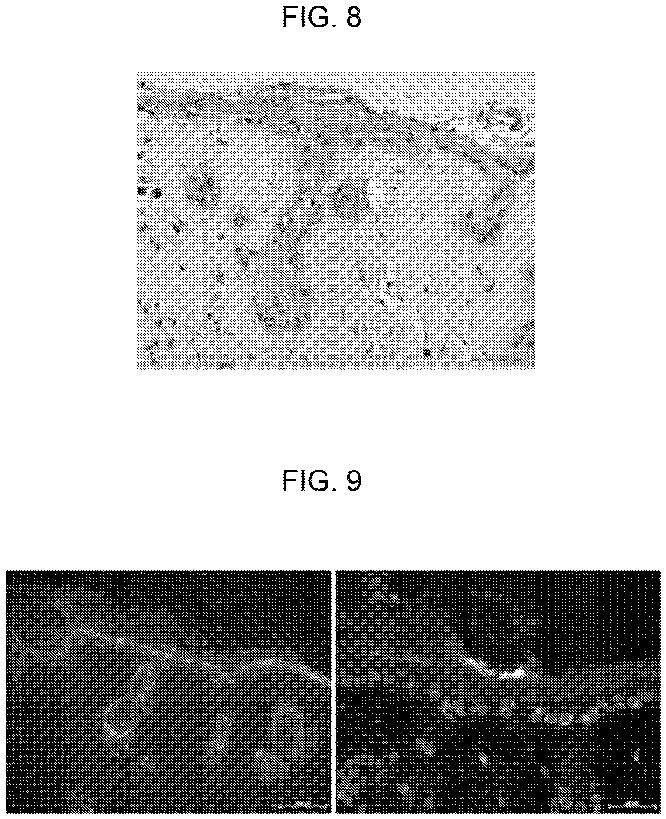

Figure 9 shows immunofluorescent images indicating that keratinocytes seeded on the dermis equivalent, proliferated and when raised at the air-liquid interface, differentiated to produce a stratified, cornified epidermis. Moreover in the upper part of dermis it is possible to observe epidermal appendages and cyst-like structure. Positive immunostaining with Keratin 10 assess the presence of a suprabasal layer in the epidermis as well as in the appendages and cyst-like structure (scale bar 100 *µ*m). P63 positive immunostaining identifies keratinocytes stem cell in the in basal layer of epidermis as well as in the appendeages and cyst-like structure. Positive immuno staining with filaggrin assess the presence of the terminal layer of epidermis (stratum corneum) while DAPI stain all kinds of cells. Skin equivalent is featured by a well-organized epidermis, which expresses the characteristic differentiation markers observed in normal human skin (scale bar 50 *µ*m).

### DETAILED DESCRIPTION OF THE INVENTION

The method for producing a totally endogenous bioengineered tissue including a first layer of connective tissue and a second layer of epithelial tissue according to the present invention comprises several steps.

In step (a), extracellular matrix (ECM) producing cells are seeded on biodegradable porous microbeads under dynamic seeding conditions, thereby obtaining microtissues. The dynamic seeding conditions comprise intermittent stirring followed by continuous stirring. Stirring preferably has a duration from 3 to 15 minutes, preferably 5 minutes, and is alternated with a rest period preferably having a duration from 15 to 60 minutes, preferably 30 minutes. Stirring and rest periods may be alternated for a total time from 3 to 9 hours, preferably 6 hours, at the end of which the cell/microspheres suspensions can be maintained under continuous stirring with a duration ranging from 7 to 12 days, preferably 9 days.

The stirring rate is preferably from 20 to 40 rpm, more preferably 30 rpm. This rate allows to maintain microbeads and cells in suspension and avoids damage induced by mechanical stress due to stirring.

The dynamic cell seeding allows a uniform distribution of the cells onto the surface of the microbeads, creates an homogeneous culturing environment, avoids microbeads aggregation and improves gas and nutrient exchange between the cell seeded microbeads and the culture medium.

5x10⁵ cells/mg of micro-carrier material are seeded on micro-beads and cultured in MEM Eagle-Earle BSS enriched with a solution of essential and non-essential amino acids (2x), 20% FBS, and glutamine 2 mM.

The extracellular matrix (ECM) producing cells are preferably fibroblasts, more preferably primary fibroblasts.

In step (b), the microtissues are placed and molded in a maturation chamber suitable to guarantee nutrient supply and provisional support to the microtissues.

In particular, the maturation chamber has a sandwich-like structure, in the middle of which a silicon mold is present. The silicon mold is provided with empty spaces (maturation space) where the microtissue assembling can take place. The maturation space can have several shapes and dimensions. The silicon mold is delimited on both the top and bottom sides by two stainless steel rigid grids characterized by a porous mesh (18 *µ*m) that is able to retain the microtissue while allowing nutrient supply and waste removal. Two polytetrafluoroethylene (PTFE) spacers are placed on the grids on both sides of the system and are fastened to each other by means of stainless steel screws, which close the system and ensure that the microtissue are retained. The system is autoclavable in each part.

The microtissue suspension is transferred from the spinner flask, where step (a) is carried out, to a 50 ml Falcon centrifuge tube and, after settling, transferred by pipetting into the maturation spaces of the silicon mold of the assembling chamber to allow the assembly.

In step (c) the microtissues are cultured in the maturation chamber, thereby obtaining the first layer of connective tissue.

In particular, the maturation chamber is placed on the bottom of a spinner flask and completely surrounded by culture medium. The spinner is operated at 50 rpm and the medium is replaced every 3 days. After 4 to 8 weeks of culture, preferably 6 weeks, the maturation chamber is opened and a totally endogenous bioengineered tissue including a first layer of connective tissue, hereinafter also referred as a dermis equivalent, is collected for histological analysis.

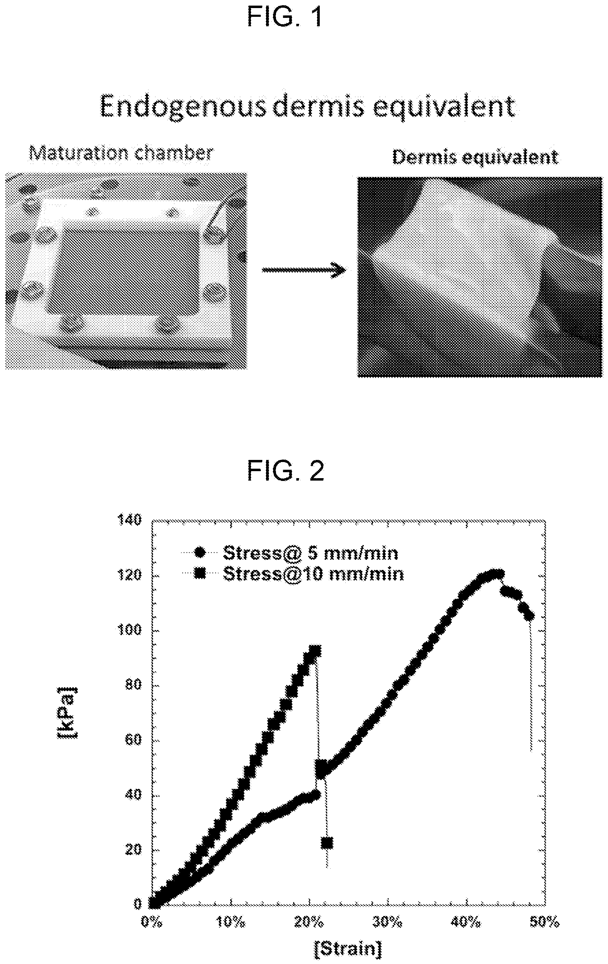

The process of building up a dermis equivalent based on the bottom-up method has the advantage that sufficient dermal microtissue can be made available with a smaller starting amount of fibroblasts. In this way a dermis equivalent 25 cm² in size is easily obtained, as may be seen in Figure 1. Moreover, it is easy to handle by comparison with known models based on the use of exogenous collagen as ECM-like structure, because it is thick (1-2 mm) and particularly robust, as demonstrated by mechanical tests shown in Figure 2.

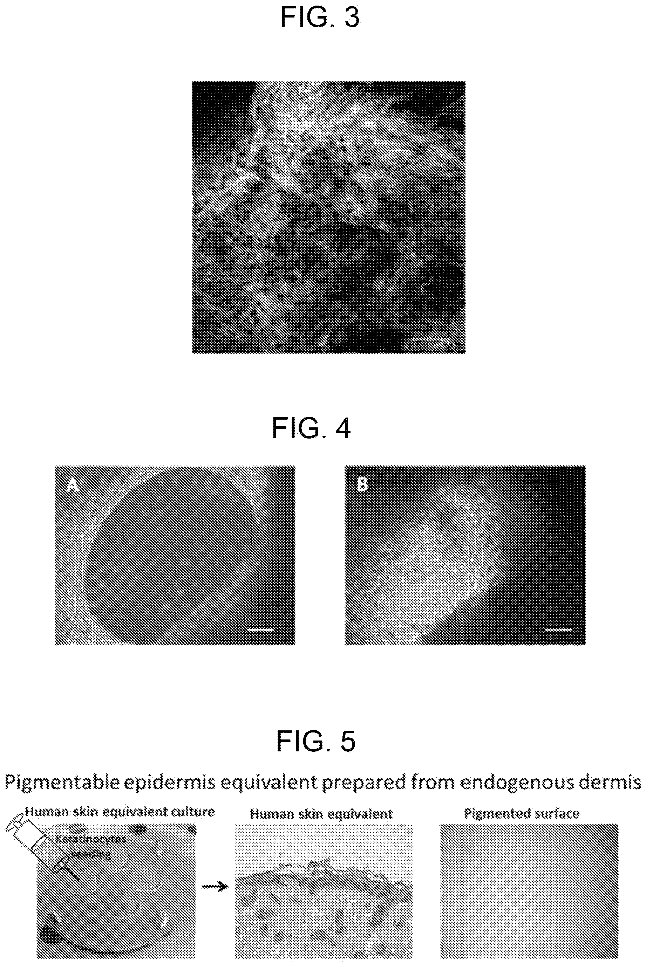

Further, the presence of a high amount of endogenous collagen (as may be observed in Figure 3) allows the further bio-fusion in a 3D dermal equivalent. The initial presence of a microscaffold has a crucial importance to promote the synthesis and organization of the ECM. By tuning the degradation rate of microscaffold present in the dermal microtissue in order to match with ECM synthesis and maturation, a 3D dermal equivalent completely made up of endogenous ECM is obtained. Moreover, the addition of ascorbic acid to the culture medium stimulates the synthesis and the secretion of appropriate ECM components. The dermis equivalent represents a connective-tissue-like layer of endogenous ECM and fibroblasts, which substantially corresponds to the native dermis. Since the fibroblasts are embedded in their own ECM they can react to an external stimulus or environmental change as they do in a native dermis.

The method of the invention preferably further comprises the step of preparing the biodegradable porous microbeads before step (a).

The microbeads are for example gelatin microbeads with a controlled degradation rate, obtained by means of the "oil-in water-in oil" (O/W/O) double emulsion technique, by mixing an aqueous solution of gelatin comprising a first surface active agent, with a hydrophobic organic solvent comprising a second surface-active agent.

The O/W/O emulsion consists of a three-phase system in which a water phase, containing a first hydrophobic phase, is dispersed in a second hydrophobic phase.

The O/W/O emulsion is prepared by mixing an aqueous solution of gelatin comprising a first surface-active agent, such as a polyethoxylated sorbitol ester, with a hydrophobic organic solvent, for example toluene, comprising a second surface active agent, for example a sorbitol ester (step (i)). The first surface active agent is preferably TWEEN 85 (sorbitan trioleate ethoxylated). The second surface active agent is preferably SPAN 85 (sorbitan trioleate).

The addition of the hydrophobic organic solvent to the aqueous phase leads to the formation of a hydrophobic phase in the form of organic solvent droplets trapped within the aqueous phase. When addition of the organic solvent leads to saturation of the aqueous solution, the latter becomes dispersed in an external hydrophobic phase of the solvent. Gelatin microbeads with entrapped organic solvents droplets are thus formed. The gelatine microbeads are subsequently solidified by cooling.

In the next step (step (ii)), the microbeads are washed with a solvent miscible with the hydrophobic solvent used for the preparation of the double emulsion which is not capable of dissolving gelatin, for example ethanol may be used when toluene is used in the emulsion. Accordingly, the droplets of hydrophobic organic solvent trapped within the microbeads are removed, thereby forming an extended network of micropores in the microbeads. After a final wash with acetone the microbeads are dried at room temperature and subsequently sieved.

In a preferred embodiment of the method, the biodegradable porous microbeads have a diameter ranging from 75 *µ*m to 150 *µ*m, more preferably 150 *µ*m.

In the next step (step (iii)), the gelatin porous microbeads are stabilized by means of a treatment with a cross-linking agent, the amount of which influences the rate of degradation of the microbeads. It is thus possible to modulate the rate of degradation of the microbeads by varying the concentration of the crosslinking agent during the stabilization, thereby synchronizing the rate of degradation of the gelatin microbeads with the rate of extracellular matrix synthesis of the cells seeded within the microbeads.

The cross-linking agent, which is preferably glyceraldehyde, is preferably used in a concentration from 2 % w/w to 6 % w/w of the microbeads weight.

Preferably, the gelatine porous microbeads may be subjected to sterilization, so as to provide for a sterilized environment for the cells which will be seeded into the microbeads. Preferably, sterilization may be carried out by means of autoclaving or gamma-ray irradiation or by sucking in ethanol solution.

In step (d), epithelial cells are seeded on the first layer of connective tissue. Epithelial cells are preferably undifferentiated keratinocyte stem cells from human biopsy tissue, preferably foreskin or breast biopsy.

In step (e), the epithelial cells seeded on the first layer of connective tissue of step (d) are cultured for 4 to 8 days in submerged conditions, thereby allowing the proliferation of the epithelial cells.

In step (f) the proliferated epithelial cells seeded on the first layer of connective tissue of step (e) are cultured above the air-liquid interface, thereby allowing differentiation of the epithelial cells and formation of the second layer of epithelial tissue.

More specifically, complete differentiation of the keratinocyte layers is achieved by a so-called "airlift culture" in KGM2 without hEGF and BPE (bovine pituitary extract) and with an increased concentration of CaCl₂. By "airlift culture" there is intended a culture where the height of the nutrient medium level is adapted exactly to the height of the human dermal equivalent whereas the keratinocytes or cell layers formed by the keratinocytes lie above the nutrient medium level and are not covered by the nutrient medium, i.e. cultivation takes place at the air/nutrient medium interface, the cultures being fed from below. To this end, the skin models, for example, can be maintained in the maturation chamber in the open configuration, accommodated in a Petri dish.

The medium is introduced into the Petri dishes to such a level that it does not completely cover the metallic grid, instead a liquid collar is present around the base of the skin models (air/liquid interface). The duration of the airlift culture may be varied as required by the expert. Typically, it is about 2 weeks. During this period, a totally endogenous bioengineered tissue including a first layer of connective tissue, in particular dermis, and a second layer of epithelial tissue, in particular epidermis, is developed. Hereinafter it will also be referred to as "skin equivalent".

The dermal layer of the skin equivalent is formed by fibroblasts and neo-formed organized endogenous ECM including collagen, elastin and all components present in native dermis. The epidermal layer is fully differentiated and is well anchored to the dermis equivalent, includes keratinocytes and melanocytes as cell population and expresses all epidermal differentiation markers. Further, the epidermal layer of the skin equivalent comprises at least one proliferative cell layer, a few differentiating cell layers and at least one keratinized cell layer. By immunostaining analyses it is possible to detect Stratum basale, Stratum spinosum, Stratum granulosum and Stratum corneum and a basal membrane consisting of the characteristic BM proteins, such as laminin and collagen IV, and including P63 positive keratinocytes, being present between the dermal layer and the epidermal layer and, in addition, skin-typical proteins, such as, involucrin, collagen IV, fibronectin, laminin, filaggrin, cytokeratin 10, cytokeratin 14 and, in particular, elastin being expressed.

In view of its complexity, the skin equivalent can be specifically used for tackling various problems in the chemical/pharmaceutical industry and in the cosmetics industry. Since an endogenous ECM is present, it is possible to study not only the cellular response but also the ECM response in terms of change in assembly as well as composition to a chemical, pharmaceutical or cosmetic product. The product can be tested for its effectiveness, unwanted side effects (e.g. irritation, toxicity and inflammation) or allergenic effects, or the compatibility of substances. In addition, the skin equivalent may also be used, for example, for studying the absorption, transport and/or penetration of substances. It is also suitable for studying other agents (physical quantities), such as light or heat, for studying photoxicity, i.e. the damaging effect of light of different wavelengths on cell structures as well as on ECM organization. Moreover the skin equivalent may also be used for treating patients suffering from a wound to the skin, for example, burn patients. The skin equivalent may be applied to the wound, for example, by transplanting or grafting.

The method for determining the effect of a chemical substance or an agent on skin of the present invention comprises the steps of:
- bringing into contact the chemical substance or agent with the totally endogenous bioengineered tissue disclosed above; and
- determining the effect of the contact between the chemical substance or agent and the totally endogenous bioengineered tissue.

Preferably the chemical substance may be a drug or a cosmetic and the agent may be light or heat, radioactivity, sound, electromagnetic radiation, electrical fields.

In particular, the effects of irradiation with UVA may be studied and the organization of endogenous structural proteins such as collagen and elastin can be observed and measured by means of multiphoton microscopy MPM.

The tissue equivalent may also be used for studying wound healing processes. By inducing a wound on the dermis equivalent, the cells are able to remodel their own ECM and to synthesize neo-ECM in order to close the wound, in this way a process of self-repair occurs (see Figure 4) and the effects of wounds on cells as well as ECM structure or on gene expression can be studied.

### Examples

Example 1: keratinocyte, melanocyte and fibroblast extraction from foreskin

Foreskin samples have been obtained from reduction surgery and treated in order to remove all fat, reticular dermis and tissue remnants from the bottom side, resulting in processing for cell extraction only papillary dermis (about 0.3 mm in thickness). The "purified" skin was then rinsed in PBS and cut into strips of about 3 mm width. The strips were completely covered with Dispase II (Gibco) solution (1.8 U/mL; about 5-10 ml for 6 cm² of skin) and incubated over night for 16 h to 18 h. After 16 h the epidermis begins to detach from dermis. By adding PBS, the dispase reaction is stopped. Epidermis detached from the dermis can easily be separated by means of tweezers. Epidermis and dermis can be separately treated in order to extract keratinocytes from the former and papillary fibroblasts from the latter. To extract papillary fibroblasts from dermis, it is firstly necessary to detach endothelial cells by scraping eight times both surfaces of dermal piece in PBS solution. The dermis deprived from endothelial cells is cut in small pieces and put in 30 ml collagenase A solution (ROCHE) at a concentration of 2 mg/ml for 40 minutes at 37°C. The reaction of collagenase is stopped by adding FBS, the suspension centrifuged 5 min at 1200 rpm and then re-suspended in a little volume of EMEM (Eagle's BSS Minimum Essential Medium) containing 20% FBS, 100 mg/mL L-glutamine, 100 U/mL penicillin/streptomycin, and 0.1 mM Non Essential Amino Acids. Finally, the suspension was transferred in a Petri dish in order to allow the migration of papillary fibroblasts from the collagenase treated dermis pieces to the surface of the Petri. The migration occurred after three days. When confluence is reached, papillary fibroblasts are trypsinized and collected. To extract keratinocytes and melanocytes, epidermis was cut into small pieces and then transferred into a pre-heated solution of Trypsin/EDTA (5 mL 0.05%) for 5 min at 37°C, vortexed shortly every 1-2 min. The enzymes reaction is stopped by adding 1 mL FBS, the solution is suspended for 5 min to separate cell clumps. Then the solution is filtered by using 70-100 *µ*m strainer and 10 ml of PBS is added in the strainer in order to collect all the cells. The cells suspension is centrifuged 5 min at 1200 rpm. The cells are re-suspended in culture media and counted. Finally the cells (a mixed population including keratinocytes and melanocytes) are seeded into cells flask at concentration of 8*10³ cells per cm². According to literature data the keratinocytes are the predominant cell type, holding a ratio of 40 keratinocytes to 1 melanocyte. The medium has to be changed 4 h after the seeding of the extracted cells. Then the following medium's change will occur after 3 days. The culture medium used is crucial in order to obtain subcultures of undifferentiated keratinocytes. A serum-free medium (KGM™-2, Promocell) is used. The medium was enriched by adding calcium chloride, BPE (bovine pitutary extract), hEGF, insulin (recombinant human), hydrocortisone, GA-1000 (gentamicin, amphotericin-B), epinephrine, transferrin. The formulation was optimized for initial seeding of 5000 cells per cm² up to sub-confluence. In this condition feeder-layer, ECM substrates or other substances to promote cells adhesion are not necessary.

### Example 2: Fabrication of an endogenous human dermis equivalent

Dermal micro-tissue have been produced by seeding human dermal papillary fibroblasts (HDPF) at passages 2-9 on Gelatin Porous Microcarriers (GPM) having tunable degradation rate. Cultivation was initiated by inoculating HDF and GPM at 10 cells/bead in a spinner flask bioreactor. The culture suspension was stirred intermittently at 30 rpm (5 min stirring and 30 min static incubation) for the first 6h post-inoculation for cell adhesion, and then continuously agitated at 30 rpm. The growth medium (EMEM with NEEA, glutamine, Pen/Strep and 20% of FBS) was replenished on the first day and every 2 days until the end of experiments (9 days in total). From the 4^{th} day 50 *µ*g/ml of ascorbic acid was added. For the production of the 3D endogenous human dermis equivalent, the dermal micro-tissue suspension was transferred into the maturation chamber where a bio-sintering process takes place allowing the molding in a square-shaped tissue equivalent (1 mm in thickness, 5x5 cm) (Figure 1). Maturation of the 3D-human dermal equivalent was carried on up to 8 weeks of culture by placing the maturation chamber on the bottom of a spinner flask operated at 60 rpm. The medium was changed every 3 days and at each medium change ascorbic acid was added. The microscaffold crossliking was adjusted in order to match the microcarriers's degradation with collagen synthesis and maturation in order to obtain ECM able to balance both scaffold's mass loss and cell traction in a limited time window. Histological and morphological analyses showed that endogenous ECM was present in the 3D dermis equivalent realized. In addition second harmonic generation imaging in Figure 3 confirms the presence of neo-synthesized collagen. The dermis equivalent (Figure 1) produced can be handled without any breakage. To test its tensile strength bone-dog-shaped samples have been obtained to perform the mechanical tests. The samples were 1 mm in thickness, 5 mm in width and 22 mm in length. The sample was placed between mechanical grips and mechanical tests were performed by means of Instron 5566. The test was carried out under displacement control with two speeds such as 5 mm/min and 10 mm/min and by using a loading cell of 2.5 kN. Stress-Strain curves are reported in Figure 2. Elastic "modulus (E), maximum stress (σ) and maximum strain (ε) have been measured at both deformation rates. The mechanical test shows that the stiffness of the sample increases with the deformation rate (Figure 2).

| mm/min | Eₜₐₙ | ∑ₘₐₓ | εₘₐₓ |
|---|---|---|---|
| 5 | 0.173 | 0.120 | 40 |
| 10 | 0.260 | 0.90 | 20 |

### Example 3: Production of a whole skin equivalent

In order to produce a whole skin equivalent an epidermal layer has been developed on the endogenous dermis equivalent. To facilitate the initial adhesion of extracted epidermal cells such as keratinocytes and melanocytes on the dermis-equivalent it is possible to add drop by drop on the human dermis equivalent surface 50 *µ*l of a solution of human fibronectin (Sigma Aldrich, 50 *µ*g/ml in sterile balanced salt solution) and wait for 45 minutes to let it dry. Meanwhile, cells in Petri dishes have been washed 3 or 4 times with PBS/EDTA 0.01 M and then trypsinized with trypsin/EDTA for 5 min at 37°C to obtain a single cell suspension. Once cells have completely detached from the plate, they have been gently re-suspended in keratinocyte media (KBM 2 medium contain 5% FBS, 0.06 mM CaCl₂, hEGF (0.1 *µ*g/500 ml medium) and BPE (15 mg/500 ml medium), cells have been counted and an appropriate volume of culture medium has been added in order to obtain a cells suspension at density of 8*10⁶ cells/ml. The maturation chamber, where maturation of endogenous human dermis equivalent took place, was open and the dermal equivalent remained on the metallic grid accommodated in a Petri-dish where it was possible to adjust the level of the culture medium according to the culture conditions required (sub-merged and air liquid interface). 50 *µ*l of cell suspension were dropped on the dermis equivalent surface pre-treated with fibronectin solution. The submersed culture took from 1 to 3 days, whereby the KBM 2 medium containing 5% FBS, 0.06 mM CaCl₂, hEGF (0.1 *µ*g/500 ml medium) and BPE (15 mg/500 ml medium) was replaced on a daily basis with fresh medium. The submersed cultivation continued for 2 or 3 days in KBM medium containing 2% FBS, 0.06 mM CaCl₂ hEGF, and BPE. At last air-liquid interface cultivation was performed by lowering the level of the medium that is exactly adapted to the height of the dermal layer, while the keratinocytes or the layer formed by the keratinocytes are not covered by the medium allowing vertical differentiation of keratinocytes and epidermis stratification. In this phase the medium used was KBM without hEGF and BPE and with 1.88 mM of CaCl₂. The air-lift cultivation is continued for at least 12 to 14 days. At the end of the culture a well stratified epidermis is formed and keratinocytes differentiation markers have been detected (Figure 9).

### Example 4: Detection of dermal-epidermal junction in the full skin model

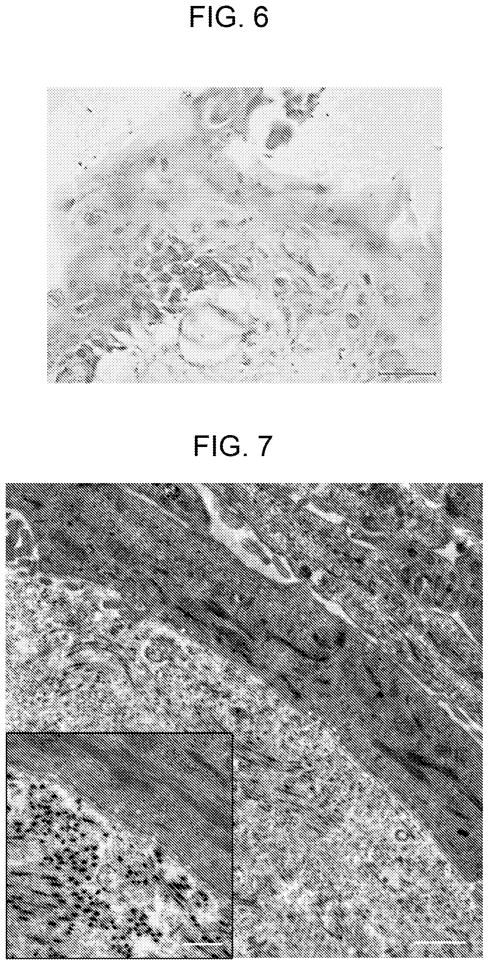

The fine structure of the dermal-epidermal junction has been investigated by means of transmission electron microscopy (TEM). To this end the samples were embedded in epoxy resin and stained with uranyl acetate and lead citrate. Ultrathin sections were examined by TEM (FEI Tecnai G2) at nominal magnifications and 100 kV acceleration voltage. As shown in Figure 7 the dermal layer of the skin equivalent is rich in collagen bundles having cross-striated patterns. Moreover, just below the basal layer many smaller collagen fibrils, such as collagen IV and VII are present and are localized close to the lamina densa and are 50-80 nm in thickness. In the same image it is possible to observe the melanocytes correctly localized along the basal membrane. In particular melanosomes and single pigments of melanin in the dendritics elongation of the melanocytes are well evident. At higher magnification (see inset) a detail of the basal membrane zone is reported. It highlights hemidesmosomes composed of an the electron-dense inner plaque into which intermediate filaments (tonofibrills) are inserted, and an outer plaque that lies on the plasma membrane. Since hemidesmosomes are junctions constituting the main adhesion units of the basement membrane zone, the great quantity of hemidesmosomes found in our skin model, demonstrated the good adhesion and cross-talking existing between dermis and epidermis. Finally, lamina lucida having a thickness of 30-50 nm is correctly present between the plasma membrane of keratinocytes and lamina dense.

### Example 5: Detection of melanocytes

In order to assess the presence of melanocytes in our model, a histochemical assay, such as Fontana Masson Staining has been used. As shown in Figure 6 melanocytes are homogeneously distributed along the basal layer of keratinocytes. Moreover melanin (dark stained) was transferred also in keratinocytes on the upper layer, this phenomenon indicates a correct functionality of the melanin synthesis and transfer pathway. The number of melanocytes present in the skin model is similar to that found in native human skin.

From an analysis of the features of the method of the present invention, the resulting advantages are apparent.

In particular, the skin equivalent that the method of the present invention allows to obtain has optimal mechanical properties in comparison to the prior art skin equivalents.

In virtue of the fact that fibroblasts are embedded in their own ECM, the dermal layer of the skin equivalent of the present invention is sufficiently thick and allows for the full development of an epidermal layer that perfectly mimics structural organization, function and composition of native epidermis.

Moreover, in virtue of the fact that the skin equivalent is totally endogenous, dermal and epidermal cells therein exchange molecular signals which are virtually identical to those in native tissues.

The skin equivalent presents a fully differentiated epidermis, which is well anchored to the dermis equivalent including keratinocytes and melanocytes and expressing epidermal differentiation markers.

Further, the dermal layer which is part of the skin equivalent of the invention undergoes very little shrinkage due to the low capacity of papillary fibroblasts to contract the collagen fibers. In particular, it is only subjected to defined, slight shrinkage in vertical direction, while shrinkage in a horizontal direction is prevented. As a result, a skin equivalent with a defined diameter and a uniform surface is obtained. In tests on substances for pharmacological and/or cosmetic effects, the uniform size and uniform properties of the full skin model used as test surface permit higher quality results and more reproducible test results.

The method of the invention for the production of a skin equivalent may advantageously be modified so that other cell types, such as, induced and non-induced precursor cells of Langerhans cells, Langerhans cells and other immune cells can be added to epidermal cells. The cells may be of human or other animal origin.

The method of the invention may also be modified to produce other kinds of epithelium such as bronchial epithelium, intestine epithelium and cervix epithelium.

## Claims

1. A method for producing a totally endogenous bioengineered tissue including a first layer of connective tissue and a second layer of epithelial tissue comprising the steps of:
(a) seeding extracellular matrix (ECM) producing cells on biodegradable porous microbeads under dynamic seeding conditions, thereby obtaining microtissues, said dynamic seeding conditions comprising intermittent stirring followed by continuous stirring;
(b) placing and molding the microtissues in a maturation chamber suitable to guarantee nutrient supply and provisional support to the microtissues;
(c) culturing the microtissues in the maturation chamber, thereby obtaining the first layer of connective tissue;
(d) seeding epithelial cells on the first layer of connective tissue;
(e) culturing the epithelial cells seeded on the first layer of connective tissue of step (d) for 4 to 8 days in submerged conditions, thereby allowing the proliferation of the epithelial cells;
(f) culturing the proliferated epithelial cells seeded on the first layer of connective tissue of step (e) above the air-liquid interface, thereby allowing differentiation of the epithelial cells and formation of the second layer of epithelial tissue.

2. The method of claim 1, wherein in step (a) intermittent stirring is carried out for a period from 3 hours to 9 hours and continuous stirring is carried out for a period from 7 to 12 days, intermittent stirring comprising stirring for a period from 3 to 15 minutes alternated with a rest period from 15 to 60 minutes.

3. The method of claim 1, wherein the maturation chamber comprises a top and a bottom grid, and a silicon mold including empty spaces placed between the grids.

4. The method of claim 1, wherein the extracellular matrix (ECM) producing cells are fibroblasts.

5. The method of claim 1, further comprising the step of preparing the biodegradable porous microbeads before step (a), said step of preparing the biodegradable porous microbeads comprising the steps of:
(i) preparing an oil-in-water double emulsion mixing an aqueous solution of gelatin comprising a first surface active agent with a hydrophobic organic solvent comprising a second surface active agent, thereby obtaining gelatin microbeads;
(ii) removing the hydrophobic organic solvent from the gelatin microbeads by washing with a solvent miscible with said hydrophobic organic solvent, thereby obtaining porous gelatin microbeads,
(iii) treating the porous gelatin microbeads with a crosslinking agent.

6. The method of claim 5, wherein the crosslinking agent is in an amount from 2 % w/w to 6 % w/w of the microbeads weight.

7. The method of claim 5, wherein the crosslinking agent is glyceraldehyde.

8. The method of claim 1, wherein the epithelial cells are undifferentiated keratinocyte stem cells.

9. A method for determining the effect of a chemical substance or an agent on skin comprising the steps of:
- bringing into contact the chemical substance or agent with a totally endogenous bioengineered skin obtained by the method of claim 1; and
- determining the effect of the contact between the chemical substance or agent and the totally endogenous bioengineered skin.

## Patentansprüche

1. Verfahren zur Herstellung eines gänzlich endogen biotechnologisch hergestellten Gewebes einschließlich einer ersten Schicht aus Bindegewebe und einer zweiten Schicht aus Epithelgewebe, das die Schritte umfasst:
(a) Säen von Zellen, die extrazelluläre Matrix (ECM) herstellen, auf biologisch abbaubaren porösen Mikroperlen (microbeads) unter dynamischen Anzuchtbedingungen, um dadurch Mikrogewebe zu erhalten, wobei die dynamischen Anzuchtbedingungen Intervallrühren gefolgt von kontinuierlichem Rühren umfassen;
(b) Anordnen und Formen der Mikrogewebe in einer Reifungskammer, die geeignet ist, Nährstoffversorgung und vorläufige Unterstützung der Mikrogewebe zu gewährleisten;
(c) Züchten der Mikrogewebe in der Reifungskammer, um dadurch die erste Schicht aus Bindegewebe zu erhalten;
(d) Säen von Epithelzellen auf die erste Schicht aus Bindegewebe;
(e) Züchten der auf die erste Schicht aus Bindegewebe gesäten Epithelzellen aus Schritt (d) für 4 bis 8 Tage unter submersen Bedingungen, um dadurch die Proliferation der Epithelzellen zu ermöglichen;
(f) Züchten der auf die erste Schicht aus Bindegewebe gesäten proliferierten Epithelzellen aus Schritt (e) oberhalb der Grenzfläche zwischen Luft und Flüssigkeit, um dadurch die Differenzierung der Epithelzellen und die Bildung der zweiten Schicht aus Epithelgewebe zu ermöglichen.

2. Verfahren nach Anspruch 1, wobei in Schritt (a) das Invervallrühren für einen Zeitraum von 3 Stunden bis 9 Stunden und das kontinuierliche Rühren für einen Zeitraum von 7 bis 12 Tagen durchgeführt wird, wobei das Intervallrühren das Rühren für einen Zeitraum von 3 bis 15 Minuten im Wechsel mit einer Ruhephase von 15 bis 60 Minuten umfasst.

3. Verfahren nach Anspruch 1, wobei die Reifungskammer ein oberes und ein unteres Gitter, und eine Siliziumform, die Leerräume, die zwischen den Gittern angeordnet sind, umfasst.

4. Verfahren nach Anspruch 1, wobei die Zellen, die extrazelluläre Matrix (ECM) herstellen, Fibroblasten sind.

5. Verfahren nach Anspruch 1, die vor Schritt (a) zudem den Schritt des Herstellens der biologisch abbaubaren porösen Mikroperlen umfasst, wobei das Herstellen der biologisch abbaubaren porösen Mikrokugeln die Schritte umfasst:
(i) Herstellen einer Öl-in-Wasser Doppelemulsion durch Mischen einer wässrigen Gelatinelösung, die eine erste oberflächenaktive Substanz umfasst, mit einem hydrophoben organischen Lösungsmittel, das eine zweite oberflächenaktive Substanz umfasst, um dadurch poröse Gelatinemikroperlen zu erhalten;
(ii) Entfernen des hydrophoben organischen Lösungsmittels von den Gelatinemikroperlen durch Waschen mit einem mit dem hydrophoben organischen Lösungsmittel mischbaren Lösungsmittel, um dadurch poröse Gelatinemikroperlen zu erhalten;
(iii) Behandeln der porösen Gelatinemikroperlen mit einem Vernetzungsmittel.

6. Verfahren nach Anspruch 5, wobei das Vernetzungsmittel in einer Menge von 2 % w/w bis 6 % w/w des Gewichts der Mikroperlen vorliegt.

7. Verfahren nach Anspruch 5, wobei das Vernetzungsmittel Glycerinaldehyd ist.

8. Verfahren nach Anspruch 1, wobei die Epithelzellen undifferenzierte Keratinozytenstammzellen sind.

9. Verfahren zur Bestimmung der Wirkung einer chemischen Substanz oder Mittels auf Haut, das die Schritte umfasst:
- Inkontaktbringen der chemischen Substanz oder des Mittels mit einer gänzlich endogen biotechnologisch hergestellten Haut, die durch das Verfahren nach Anspruch 1 erhalten wurde; und
- Bestimmen der Wirkung des Kontaktes zwischen der chemischen Substanz oder des Mittels und der gänzlich endogen biotechnologisch hergestellten Haut.

## Revendications

1. Procédé de production d'un tissu totalement endogène produit par génie biologique comprenant une première couche de tissu conjonctif et une seconde couche de tissu épithélial comprenant les étapes suivantes :
(a) l'ensemencement de cellules productrices de matrice extracellulaire (MEC) sur des microbilles poreuses biodégradables dans des conditions d'ensemencement dynamique, obtenant de cette façon des microtissus, lesdites conditions d'ensemencement dynamique comprenant une agitation intermittente suivie d'une agitation continue ;
(b) la disposition et le moulage des microtissus dans une chambre de maturation appropriée pour garantir un apport en nutriments et un support provisoire aux microtissus ;
(c) la culture des microtissus dans la chambre de maturation, obtenant de cette façon la première couche de tissu conjonctif ;
(d) l'ensemencement de cellules épithéliales sur la première couche de tissu conjonctif ;
(e) la culture des cellules épithéliales ensemencées sur la première couche de tissu conjonctif de l'étape (d) pendant 4 à 8 jours dans des conditions submergées, permettant de cette façon la prolifération des cellules épithéliales ;
(f) la culture des cellules épithéliales qui ont proliféré ensemencées sur la première couche de tissu conjonctif de l'étape (e) au-dessus de l'interface air-liquide, permettant de cette façon la différenciation des cellules épithéliales et la formation de la seconde couche de tissu épithélial.

2. Procédé selon la revendication 1, dans lequel dans l'étape (a) l'agitation intermittente est réalisée pendant une période de 3 heures à 9 heures et l'agitation continue est réalisée pendant une période de 7 à 12 jours, l'agitation intermittente comprenant une agitation pendant une période de 3 à 15 minutes en alternance avec une période de repos de 15 à 60 minutes.

3. Procédé selon la revendication 1, dans lequel la chambre de maturation comprend une grille supérieure et une grille inférieure, et un moule en silicium comprenant des espaces vides disposés entre les grilles.

4. Procédé selon la revendication 1, dans lequel les cellules productrices de matrice extracellulaire (MEC) sont des fibroblastes.

5. Procédé selon la revendication 1, comprenant en outre l'étape de préparation des microbilles poreuses biodégradables avant l'étape (a), ladite étape de préparation des microbilles poreuses biodégradables comprenant les étapes suivantes :
(i) la préparation d'une émulsion double huile dans eau en mélangeant une solution aqueuse de gélatine comprenant un premier agent tensioactif avec un solvant organique hydrophobe comprenant un second agent tensioactif, obtenant de cette façon des microbilles de gélatine ;
(ii) le retrait du solvant organique hydrophobe des microbilles de gélatine par lavage avec un solvant miscible avec ledit solvant organique hydrophobe, obtenant de cette façon des microbilles poreuses de gélatine ;
(iii) le traitement des microbilles poreuses de gélatine avec un agent de réticulation.

6. Procédé selon la revendication 5, dans lequel l'agent de réticulation est dans une quantité de 2 % p/p à 6 % p/p du poids des microbilles.

7. Procédé selon la revendication 5, dans lequel l'agent de réticulation est le glycéraldéhyde.

8. Procédé selon la revendication 1, dans lequel les cellules épithéliales sont des cellules souches indifférenciées de kératinocytes.

9. Procédé de détermination de l'effet d'une substance chimique ou d'un agent sur la peau comprenant les étapes suivantes :
- la mise en contact de la substance chimique ou de l'agent avec une peau totalement endogène produite par génie biologique obtenue par le procédé selon la revendication 1 ; et
- la détermination de l'effet du contact entre la substance chimique ou l'agent et la peau totalement endogène produite par génie biologique.
